# EUROPEAN PATENT SPECIFICATION

(11) **EP 3 597 101 B1**
(45) Date of publication and mention of the grant of the patent: **17.11.2021**
(21) Application number: 17901305.7
(22) Date of filing: 15.10.2017
(51) Int. Cl.: A61B 5/0215, A61M 25/00

(54) **INTRAVASCULAR PRESSURE MEASURING CATHETER HAVING A MOUNTING BASE**
INTRAVASKULÄRER DRUCKMESSKATHETER MIT BEFESTIGUNGSSOCKEL
CATHÉTER DE MESURE DE PRESSION INTRAVASCULAIRE COMPORTANT UNE BASE DE MONTAGE

(30) Priority: 15.03.2017 CN 201710154748
(43) Date of publication of application: 22.01.2020
(73) Proprietor: Shenzhen Insight-Med Co., Ltd., Shenzhen, Guangdong 518000 (CN)
(72) Inventor: SONG, Liang, Shenzhen Guangdong 518000 (CN); CHEN, Lili, Shenzhen Guangdong 518052 (CN)
(74) Representative: Isern Patentes y Marcas S.L.
(86) International application number: PCT/CN2017/106230
(87) International publication number: WO 2018/166195

(56) References cited:
- WO-A1-2016/192958
- WO-A2-2016/065227
- CN-A- 104 302 345
- CN-A- 105 769 153
- CN-A- 106 456 018
- CN-U- 205 866 735
- JP-A- H07 231 879
- JP-A- 2000 287 944
- US-A1- 2016 081 564
- US-A1- 2016 199 003

## Description

### Field of Invention

The present invention relates to an intravascular pressure measuring catheter, and in particular, to an intravascular pressure measuring catheter having a mounting base.

### BACKGROUND OF INVENTION

For many cardiovascular diseases such as the coronary heart disease, stenosis of blood vessels (such as stenosis of blood vessels caused by vascular plaques) can affect the normal supply of blood, and blockage of blood vessels may even be caused when stenosis is severe, which may lead to serious lesions such as myocardial infarction. Percutaneous coronary intervention (PCI) is currently an effective treatment for this type of disease. In a traditional method for determining whether interventional therapy (such as angioplasty or stenting) is to be performed, doctors and the like usually visualize the degree of coronary artery stenosis through coronary angiography. However, this traditional determining method cannot well help doctors make accurate judgments, and may lead to over-treatment by the doctors due to misjudgment.

In recent years, to more accurately determine whether patients really need interventional therapy, the use of Fractional Flow Reverse (FFR) to assess the degree of stenosis obstruction of blood flow through blood vessels has been increasingly applied and promoted. FFR is defined as a ratio of a maximum blood flow in a stenosed artery to a normal maximum blood flow. To calculate FFR of a given stenosis (that is, a position at which a vascular stent may be placed) in the blood vessel, blood pressure readings of a distal side of the stenosis (for example, downstream of the stenosis, away from the aorta) and a proximal side of the stenosis (for example, upstream of the stenosis, near the aorta) need to be measured and collected respectively. Clinical studies have shown that the higher the degree of stenosis, the lower the FFR value. Whether the FFR value is less than an evaluation value (for example, 0.8) may be used as a useful criterion for a doctor to determine whether or not interventional therapy can be applied to such patients. The validity of this criterion has been confirmed by many large clinical studies (such as FAME clinical studies) in Europe and America.

At present, pressure sensing catheters with rapid exchange (RX) port have been used in recent years in a method for measuring intravascular blood pressure to obtain FFR values of vascular stenosis. In such a pressure sensing catheter, a distal part of the pressure sensing catheter has a lumen for threading a guidewire. The distal part is sleeved on the guidewire, so that the pressure sensing catheter can be moved to a predetermined position along the guidewire. Before coronary intervention, the pressure sensing catheter passes through the distal and proximal side of the stenosis to record distal and proximal blood pressure respectively. In this way, the FFR value of the stenosis can be calculated.

State of the art intravascular pressure measuring catheters are for instance described in JP2000287944A, WO2016/192958A1, JPH07231879A and WO2016/065227A2.

### SUMMARY OF INVENTION

### Problems to be resolved in the present invention

However, an existing pressure sensing catheter needs to enter the body for pressure measuring at different positions. Therefore, the pressure sensing catheter may pass through complex and varied blood vessels, such as coronary arteries of the heart. In this case, the pressure sensing catheter is, for example, inevitably squeezed and deformed when moving in the blood vessels along a guidewire. If a pressure sensor (especially a sensing portion) provided in the pressure sensing catheter is also squeezed, for example, it may cause adverse influence on the measuring accuracy or precision of the pressure sensing catheter.

### Technical means for resolving the problems

The present invention is accomplished in view of the state of the prior art and aims to provide an intravascular pressure measuring catheter having a mounting base that can effectively improve measurement accuracy or precision of the pressure sensing catheter.

For this, the present invention provides an intravascular pressure measuring catheter according to claim 1.

The sensing portion of the pressure sensor is enabled not to be in contact with the mounting base. In this case, even if the intravascular pressure measuring catheter enters complex and varied blood vessels, it can restrain contact between the pressure sensor, especially the sensing portion of the pressure sensor, and the main body of the pressure measuring catheter. In this way, for example, the influence of the bending deformation of the pressure measuring catheter (especially the distal sleeve) on the pressure measuring result of the pressure sensor can be effectively restrained, thereby improving the measuring precision of the intravascular pressure measuring catheter.

In addition, in the intravascular pressure measuring catheter the lead portion is electrically connected to a conducting wire which is extending along the proximal portion, so as to transmit the blood pressure signal outside the body.

In addition, in the intravascular pressure measuring catheter involved in the present invention, optionally, wherein the mounting base has a stepped portion, to which the lead portion of the pressure sensor is fixed, and wherein the sensing portion and the stepped portion have a gap therebetween. In this way, the sensing portion of the pressure sensor can be well enabled not to be in contact with the mounting base.

In addition, in the intravascular pressure measuring catheter involved in the present invention, optionally, wherein the stepped portion comprises a first surface and a second surface, and there is a height difference between the first surface and the second surface, and wherein the lead portion of the pressure sensor is fixed to the first surface, and the sensing portion of the pressure sensor and the second surface have a gap therebetween, and the first surface is a chamfer inclined towards the proximal portion. In this case, the adverse effect of deformation of the distal sleeve on the pressure sensor can be further restrained.

The mounting base is fixed to the distal sleeve. When the guidewire is threaded into the distal sleeve, the mounting base and the pressure sensor provided on the mounting base can reach the lesion position along with the distal sleeve. In addition, the mounting base may alternatively be fixed to the distal sleeve through bonding or welding.

In addition, in the intravascular pressure measuring catheter involved in the present invention, optionally, wherein the mounting base has a bottom surface configured to match the shape of an outer surface of the distal sleeve, and wherein the mounting base is fixed to the distal sleeve by the bottom surface. In this way, the mounting base can be firmly fixed to the distal sleeve.

In addition, in the intravascular pressure measuring catheter involved in the present invention, optionally, futher comprising an outer tube for at least covering the mounting base, wherein a portion of the outer tube corresponding to the sensing area is provided with an opening. In this way, the pressure sensor can be both protected by the outer tube and fully in contact with blood in the blood vessels through the opening, so that blood pressure can be more accurately measured.

The mounting base has a mounting groove and a wiring groove being disposed along a length direction of the distal sleeve and communicating with each other, and wherein the pressure sensor is arranged inside the mounting groove, and a conducting wire is arranged along the wiring groove. In this case, the mounting base can both accommodate the pressure sensor and easily guide the conducting wire drawn from the pressure sensor.

In addition, in the intravascular pressure measuring catheter involved in the present invention, optionally, wherein the width of the mounting groove is greater than the width of the pressure sensor, the length of the mounting groove is greater than the length of the pressure sensor, and the depth of the mounting groove is greater than the thickness of the pressure sensor. In this way, the mounting base can fully accommodate the pressure sensor therein, achieving a good protecting effect.

In addition, in the intravascular pressure measuring catheter involved in the present invention, optionally, wherein the lead portion has a bonding pad area, and the conducting wire is connected to the bonding pad area through welding. In this way, the strength of bonding between the conducting wire and the bonding pad area can be enhanced.

In addition, in the intravascular pressure measuring catheter involved in the present invention, optionally, wherein the hardness of the mounting base is greater than the hardness of the distal sleeve. In this way, it can ensure that the mounting base has sufficient anti-bending strength, and the mounting base is not easily deformed even when the distal sleeve is bent, thereby ensuring measuring precision of the pressure sensor provided on the mounting base. In addition, the material of the mounting base can be optionally composed of one or more of stainless steel, modified materials of stainless steel, cobalt alloy, chromium alloy, or molybdenum alloy. In addition, the material of the mounting base may alternatively be a high-strength modified polymer material.

In addition, in the intravascular pressure measuring catheter involved in the present invention, optionally, wherein the Young's modulus of the mounting base is greater than the Young's modulus of the distal sleeve. Generally, the Young's modulus reflects a physical quantity of a deformation resistance capability of a solid material. The higher the Young's modulus, the better the deformation resistance capability of the solid material. Therefore, making the Young's modulus of the mounting base greater than the Young's modulus of the distal sleeve can ensure that the mounting base has sufficient anti-bending strength. In this way, the mounting base is not easily deformed even when the distal sleeve is bent, thereby ensuring measuring precision of the pressure sensor provided on the mounting base.

In addition, in the intravascular pressure measuring catheter involved in the present invention, optionally, wherein the guidewire lumen of the distal sleeve comprises an extension segment and a bending segment. Further, the specified angle formed by the center line of the extension segment and the center line of the bending segment is greater than 0° and less than 90°. In this case, the increase in the cross-sectional area of the distal sleeve part can be restrained, thereby restraining the adverse effect of the cross-sectional area of the distal sleeve on blood pressure measuring precision.

In addition, in the intravascular pressure measuring catheter involved in the present invention, optionally, wherein the guidewire lumen comprises a receive opening located at the foremost end of the distal sleeve and a guide opening located at a side portion of the distal sleeve, and wherein the medical guidewire enters the guidewire lumen from the receive opening and extends out through the guide opening. In this way, the damage to the distal sleeve or the proximal portion caused by the medical guidewire can be restrained.

In addition, in the intravascular pressure measuring catheter involved in the present invention, optionally, wherein the guidewire lumen comprises a receive opening located at the foremost end of the distal sleeve and a guide opening located at a side portion of the distal sleeve, and wherein the medical guidewire enters the guidewire lumen from the guide opening and extends out through the receive opening. In this way, the damage to the distal sleeve or the proximal portion caused by the medical guidewire can be restrained.

### Beneficial effects of the present invention

According to the present invention, the intravascular pressure measuring catheter having a mounting base that can restrain the influence of bending deformation of the medical catheter on the pressure measuring result and improve the measuring precision of the intravascular pressure measuring catheter can be provided.

### BRIEF DESCRIPTION OF THE DRAWINGS

FIG. 1 is a three-dimensional schematic structural diagram of the intravascular pressure measuring catheter having a mounting base involved in an implementation of the present invention.
FIG. 2 is a three-dimensional schematic structural diagram of the intravascular pressure measuring catheter shown in FIG. 1 from another perspective.
FIG. 3 is a schematic diagram of the cross-sectional view of the guidewire lumen of the intravascular pressure measuring catheter.
FIG. 4 is a partial schematic diagarm of the cross-sectional view of the proximal portion of the intravascular pressure measuring catheter.
FIG. 5 is a schematic structural diagram of the pressure sensor of the intravascular pressure measuring catheter.
FIG. 6 is a three-dimensional schematic structural diagram of the mounting base of the intravascular pressure measuring catheter.
FIG. 7(a) is a partial schematic diagram of the cross-sectional view of the intravascular pressure measuring catheter, and FIG. 7(b) is a partially enlarged schematic diagram of the intravascular pressure measuring catheter having a mounting base shown in FIG. 7(a).
FIG. 8 is a three-dimensional schematic structural diagram of the distal sleeve of the intravascular pressure measuring catheter covered with an outer tube according to this implementation.

### Description of main reference numerals:

1...intravascular pressure measuring catheter, 10...distal sleeve, 20...proximal portion, 23...conducting wire, 30...mounting base, 40...pressure sensor, 41...sensing portion, 42...lead portion, 50...medical guidewire, 60...outer tube.

### DETAILED DESCRIPTION

Preferred implementations of the present invention will be described below in detail with reference to the accompanying drawings. In the following description, the same components are denoted by the same reference numerals, and the description thereof will not be repeated. In addition, the accompanying drawings are merely schematic diagrams, and the ratio of the dimensions of the components to each other or the shape of the components may be different from the actual ones.

The intravascular pressure measuring catheter having a mounting base involved in the present invention has a mounting base for providing a pressure sensor for measuring intravascular pressure, and it is ensured that the sensing portion of the pressure sensor is not in contact with the mounting base. In this case, even if the intravascular pressure measuring catheter involved in the present invention enters complex and varied blood vessels, for example, blood vessels with a high curvature, the contact between the pressure sensor, especially the sensing portion of the pressure sensor, and the main body of the pressure measuring catheter can be restrained. In this way, for example, the influence of bending deformation of the pressure measuring catheter (especially the distal sleeve) on the pressure measuring result of the pressure sensor can be effectively restrained, thereby improving the measuring precision of the intravascular pressure measuring catheter.

In the present description, the intravascular pressure measuring catheter sometimes is also referred to as "the blood pressure measuring catheter" or "the medical measuring catheter". In addition, the intravascular pressure measuring catheter involved in the present invention is applicable to measuring, for example, the blood pressure or the pressure gradient in the coronary artery of a patient, but the present invention is not limited thereto. For example, the intravascular pressure measuring catheter involved in the present invention is also applicable to measuring the blood pressure or the pressure gradient in the vein, heart valves and positions nearby, or other blood vessels, and other purposes for measuring fluid pressure in the body.

### (Pressure measuring catheter)

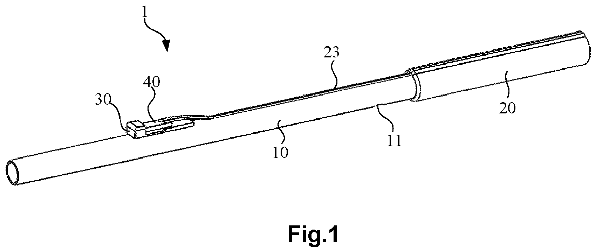

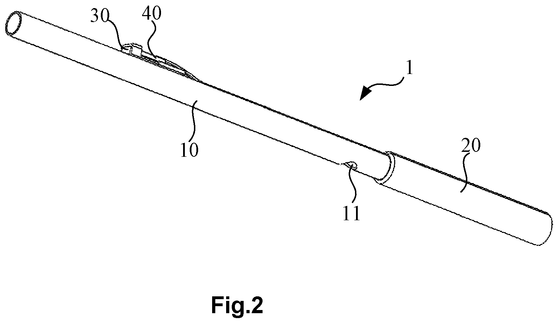

FIG. 1 is a three-dimensional schematic structural diagram of the intravascular pressure measuring catheter 1 having a mounting base according to this implementation. FIG. 2 is a three-dimensional schematic structural diagram of the intravascular pressure measuring catheter 1 shown in FIG. 1 from another angle. In FIG. 1 and FIG. 2, as an schematic, only a part of the proximal portion 20 of the intravascular pressure measuring catheter 1 close to the distal sleeve 10 is shown.

As shown in FIG. 1, the intravascular pressure measuring catheter 1 (sometimes also referred to as "the blood pressure measuring catheter 1" or "the medical measuring catheter 1") may comprise the distal sleeve 10 and the proximal portion 20 coupled (or connected) to the distal sleeve 10. In addition, the blood pressure measuring catheter 1 may further comprise a pressure sensor 40 provided on a mounting base 30. The pressure sensor 40 transmits the blood pressure signal to an external device outside the body through the conducting wire 23 which is to be described later.

In addition, as shown in FIG. 1, the mounting base 30 may be provided on the distal sleeve 10. In this case, the pressure sensor 40 provided on the mounting base 30 can also be located in an anatomical structure (for example, a coronary vessel) of a patient along with the distal sleeve 10. However, the mounting base 30 involved in this implementation is not limited to be provided on the distal sleeve 10. For example, in some examples, the mounting base 30 may alternatively be provided on the proximal portion 20. In this case, the provision of the mounting base 30 on the proximal portion 20 can reduce the cross-sectional area of the distal sleeve 10. In this way, the influence of the increase in the cross-sectional area of the distal sleeve of the blood pressure measuring catheter on the blood flow in the blood vessel can be restrained, thereby improving the measuring precision of the blood pressure measuring catheter. In addition, in some other examples, the mounting base 30 may alternatively be provided at the junction between the distal sleeve 10 and the proximal portion 20.

In this implementation, during an interventional operation performed on the patient, the blood pressure measuring catheter 1 enters the body of the patient. In this case, the distal sleeve 10 of the blood pressure measuring catheter 1 is located at a specific position within the anatomical structure of the body, for example, a stenosis of a coronary artery, and the proximal portion 20 coupled to the distal sleeve 10 extends out of the body of the patient and is connected to the external device (not shown). The external device includes, for example, a host that can be configured to process the blood pressure signal generated by the pressure sensor 40. In this implementation, such a host may have a display screen. In this case, the host may also directly display, on its display screen, the blood pressure, the blood pressure gradient, the FFR readings, or the like at a specific position of the blood vessels measured by the pressure sensor 40.

In addition, during an interventional operation, a doctor, a medical technician, or other operators, or the like may move the distal sleeve 10 by manipulating the proximal portion 20 located outside the body of the patient and coupled to the distal sleeve 10, to push the distal sleeve 10 to, for example, a lesion of a vascular stenosis in the body of the patient, and measure the blood pressure by using the pressure sensor 40, to obtain the required FFR value.

### (Distal sleeve)

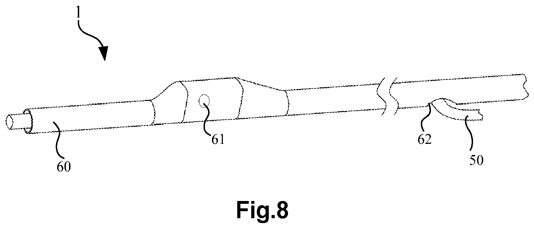

In this implementation, as shown in FIG. 1 or FIG. 2, the distal sleeve 10 is located at the front end of the blood pressure measuring catheter 1. The distal sleeve 10 has a guidewire lumen 11 (referring to FIG. 2) for slidably receiving a separate medical guidewire 50 (referring to FIG. 8 to be described later). In this case, the distal sleeve 10 may thread the separate medical guidewire 50, and may be pushed to, for example, a specific position in the body of the patient, along the medical guidewire 50. The separate medical guidewire 50 herein refers to a medical guidewire separated from the blood pressure measuring catheter 1. In other words, the medical guidewire 50 is not a necessary constituent part of the blood pressure measuring catheter 1 involved in this implementation.

In this implementation, there is no special restriction on the medical guidewire 50. For example, the medical guidewire 50 may be a guidewire commonly used in an interventional operation. In some examples, the diameter of the medical guidewire 50 is, for example, 0.1 mm to 0.6 mm, and a typical or standard diameter of the medical guidewire 50 is, for example, 0.36 mm (0.014 inches), 0.40 mm (0.016 inches), or 0.64 mm (0.025 inches). In addition, the inner diameter of the guidewire lumen 11 of the distal sleeve 10 is slightly greater than the outer diameter of the medical guidewire 50. In this case, doctors and the like can conveniently select different sizes of medical guidewires 50, as long as they can ensure that the outer diameter of the medical guidewire 50 is less than the inner diameter of the guidewire lumen 11. In this way, on the one hand, when such a medical guidewire 50 is used, the distal sleeve 10 can be easily used for threading the medical guidewire 50, and the medical guidewire 50 is caused to relatively move or slide in the guidewire lumen 11 of the distal sleeve 10. On the other hand, an increase in a cross-sectional area (especially the cross-sectional area of the distal sleeve 10) of the blood pressure measuring catheter 1 can also be restrained. In addition, to enable the medical guidewire 50 to move or slide in the guidewire lumen 11 of the distal sleeve 10, a smooth inner surface is preferably used for the guidewire lumen 11.

In addition, in some examples, the guidewire lumen 11 configured to thread the medical guidewire 50 may be provided only on the distal sleeve 10. In other words, the guidewire lumen 11 is limited to being located only at the distal sleeve 10, and separated from the proximal portion 20. In this case, because the guidewire lumen 11 of the received medical guidewire 50 is provided only on the distal sleeve 10, when the medical guidewire 50 is threaded into the blood pressure measuring catheter 1, only the distal sleeve 10 is threaded on the medical guidewire 50. In this way, different types of medical guidewires 50 can be easily used or replaced.

In addition, there is no special restriction on the linkage between the distal sleeve 10 and the proximal portion 20. For example the distal sleeve 10 may be coupled to the proximal portion 20 through bonding, or by using a connection member such as a sleeve. In addition, the distal sleeve 10 and the proximal portion 20 may be connected in a manner of continuous outer surfaces, or may be connected, for example, at staggered positions instead of in a continuous manner. In some examples, the distal sleeve 10 and the proximal portion 20 may overlap in the length direction of the blood pressure measuring catheter 1. In some other examples, the distal sleeve 10 and the proximal portion 20 may be separated from each other and coupled by using a connection sleeve.

In addition, in this implementation, there is no special restriction on the shape of the distal sleeve 10. As shown in FIG. 1, the distal sleeve 10 may be roughly in an extended circular tube shape. However, this implementation not limited thereto. The distal sleeve 10 involved in this implementation may alternatively be in an extended rectangular tube shape or elliptical tube shape, or the like. In addition, in some examples, from the perspective of easy processing and facilitation of intravascular movement, the distal sleeve 10 is preferably in the circular tube shape.

In addition, in this implementation, there is no special restriction on the length of the distal sleeve 10, as long as the distal sleeve 10 can easily thread the medical guidewire 50 and can support the medical guidewire 50 to be sliding in the guidewire lumen 11 of the distal sleeve 10. In some examples, the length of the distal sleeve 10 may be, for example, approximately 0.5 cm to 20 cm, and preferably 5 cm to 15 cm.

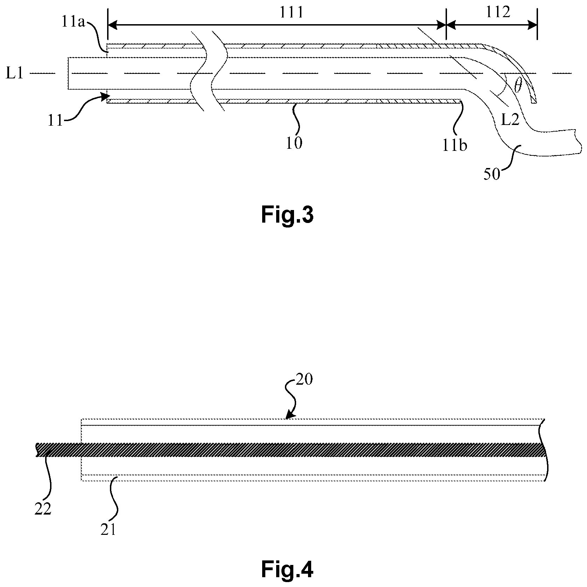

FIG. 3 is a schematic cross-sectional view of a guidewire lumen 11 of the intravascular pressure measuring catheter 1. In addition, for clear illustration, for example, figures of the mounting base 30 and the pressure sensor 40 involved in this implementation are omitted.

In this implementation, as shown in FIG. 3, the guidewire lumen 11 of the distal sleeve 10 may have a receive opening 11a and a guide opening 11b. In this implementation, as described above, the guidewire lumen 11 of the distal sleeve 10 may receive the medical guidewire 50 through the receive opening 11a, and the medical guidewire 50 slides along the guidewire lumen 11, and is guided from the guide opening 11b of the guidewire lumen 11. In this way, the blood pressure measuring catheter 1 can smoothly slide along the medical guidewire 50, to locate the distal sleeve 10 at a specific position (for example, a lesion position) in the body of the patient. In addition, in some examples, the guidewirelumen 11 of the distal sleeve 10 may alternatively receive the medical guidewire 50 through the guide opening 11b (referring to FIG. 3), so that the distal sleeve 10 can also be located at a specific location (for example, a lesion position) in the body of the patient.

In this implementation, the receive opening 11a of the guidewire lumen 11 may be provided at the foremost end of the distal sleeve 10. In this way, the medical guidewire 50 can be easily threaded into the distal sleeve 10 through the receive opening 11a. In addition, the guide opening 11b of the guidewire lumen 11 may be provided at a side portion (which may be specifically a side portion of the distal sleeve 10) of the blood pressure measuring catheter 1. In this case, the medical guidewire 50 may slide along the guidewire lumen 11 from the receive opening 11a of the guidewire lumen 11 and is drawn out from the guide opening 11b. In this way, an increase in the partial cross-sectional area of the distal sleeve 10 can be restrained, thereby restraining the adverse effect of the cross-sectional area of the distal sleeve 10 on blood pressure measurement precision.

In addition, the guidewire lumen 11 may include an extension segment 111, and a bending segment 112 communicating with the extension segment 111 (referring to FIG. 3). In this case, the extension segment 111 includes the receive opening 11a, and the bending segment 112 includes the guide opening 11b. In addition, in FIG. 3, a dotted line L1 and a dotted line L2 respectively indicate the center line of the extension segment 111 of the distal sleeve 10 and the center line of the bending segment 112. The extension segment 111 communicates with the bending segment 112 to form a through cavity, so that the separate medical guidewire 50 can be received. In other words, the medical guidewire 50 is received by using the guidewire lumen 11 including the extension segment 111 and the bending segment 112, so that the distal sleeve 10 can slide to a predetermined position (for example, a lesion position) in the body of the patient along the medical guidewire 50.

In this implementation, as shown in FIG. 3, the extension segment 111 of the guidewire lumen 11 may extend along the length direction of the distal sleeve 10. In this way, it can be ensured that the movement direction of the extension segment 111 and the movement direction the distal sleeve 10 are approximately the same. In addition, the bending segment 112 of the guidewire lumen 11 may be in a curved shape. In this case, an increase in a partial cross-sectional area of the distal sleeve 10 can be restrained, thereby restraining the adverse effect of the cross-sectional area of the distal sleeve 10 on the blood pressure measuring precision.

In addition, as shown in FIG. 3, a specified angle θ is formed by the center line L1 of the extension segment 111 and the center line L2 of the bending segment 112. From the perspective of restraining the increase in the cross-sectional area of the distal sleeve 10, the specified angle θ formed by the center line of the extension segment 111 and the center line of the bending segment 112 may be preferably greater than 0° and less than 90°. In some examples, the specified angle θ formed by the center line of the extension segment 111 and the center line of the bending segment 112 may be, for example, 20° to 60°. In addition, from the perspective of easily threading the medical guidewire 50, the specified angle θ formed by the center line of the extension segment 111 and the center line of the bending segment 112 is preferably 30° to 50°.

As described above, the medical guidewire 50 may enter from the receive opening 11a of the extension segment 111 of the guidewire lumen 11, relatively move along the extension segment 111, then enter the bending segment 112, and is finally drawn out from the guide opening 11b of the bending segment 112. In addition, in some examples, the medical guidewire 50 may alternatively enter the guidewire lumen 11 from the guide opening 11b of the bending segment 112, relatively move or slide in the guidewire lumen 11, and extend out from the receive opening 11a of the extension segment 111.

In addition, in this implementation, the medical guidewire 50 is restrained by the bending segment 112 when relatively moving along the guidewire lumen 11, so that the movement direction of the medical guidewire 50 changes accordingly. In this way, damage to the distal sleeve 10 or the proximal portion 20 caused by the medical guidewire 50 (specifically, the end of the medical guidewire 50 that first enters the guidewire lumen 11) can be restrained.

In addition, in this implementation, the medical guidewire 50 can extend or enter from a side surface (specifically, the guide opening 11b of the bending segment 112) of the distal sleeve 10. Therefore, the distal sleeve 10 and the proximal portion 20 can be coupled, for example, coaxially (for example, referring to FIG. 1). In this case, the increase in a cross-sectional area of the entire blood pressure measuring catheter 1 (especially at the junction between the distal sleeve 10 and the proximal portion 20) can be effectively restrained, so that the blood pressure measuring catheter 1 can easily move in the blood vessels, and the blood pressure measuring catheter 1 can be delivered to a narrower blood vessel in the body.

In addition, although as described above, the guidewire lumen 11 includes the extension segment 111 and the bending segment 112, the present invention is not limited thereto. For example, in some examples, the guidewire lumen 11 may be composed of only the extension segment 111 said above. In this case, the distal sleeve 10 and the proximal portion 20 may alternatively be coupled, for example, at staggered positions (not shown) instead of in a continuous manner.

In addition, in some examples, the distal sleeve 10 may be preferably optionally composed of at least one of polyimide (PI), polyester, or nylon. In this way, it can ensure that, when passing through complex bending blood vessels, the distal sleeve 10 can be deformed to adapt to the shape of the blood vessels, thereby restraining damage to the blood vessels caused by the distal sleeve 10 of the blood pressure measuring catheter 1.

### (Proximal portion)

FIG. 4 is a partial schematic cross-sectional view of the proximal portion 20 of the intravascular pressure measuring catheter. FIG. 4 shows only a part of the proximal portion 20 of the intravascular pressure measuring catheter, particularly a part close to the distal sleeve 10.

In this implementation, the proximal portion 20 is coupled to the distal sleeve 10. The proximal portion 20 is located at a rear end (relative to the distal sleeve 10 located at the front end of the blood pressure measuring catheter 1) of the blood pressure measuring catheter 1 (referring to FIG. 1). As shown in FIG. 4, the proximal portion 20 may include a proximal outer tube 21 and a core shaft 22 provided in the proximal outer tube 21. In addition, in the proximal portion 20, a medium or protection glue may be filled between the proximal outer tube 21 and the core shaft 22.

It should be noted that, the proximal outer tube 21 and the core shaft 22 shown in FIG. 4 are not necessarily drawn proportionally. For example, in some examples, the diameter of the core shaft 22 may be greater. In addition, in some other examples, the core shaft 22 may fill the lumen of the proximal outer tube 21.

In addition, the core shaft of the proximal portion 20 may, for example, connect the distal sleeve 10 to an external device (not shown) located outside the body. In addition, the conducting wire 23 (referring to FIG. 1) connected to the pressure sensor 40 may be provided in the core shaft 22 of the proximal portion 20, so that the conducting wire 23 can be drawn to the external device outside the body along the core shaft 22. In this way, the blood pressure signal generated by the pressure sensor 40 can be transmitted to the external device. In addition, in some examples, the conducting wire 23 may alternatively be arranged between the core shaft 22 and the proximal outer tube 21. In this case, the conducting wire 23 can also transmit the blood pressure signal generated by the pressure sensor 40 to the external device.

In some examples, the proximal outer tube 21 may be composed of, for example, a stainless steel hypotube. In addition, the proximal portion 20 may also be composed of other materials, such as nitinol, nylon, or plastics. In addition, in some examples, the core shaft 22 may run through the proximal outer tube 21 along the length direction of the proximal portion 20.

In addition, one end of the proximal portion 20 is coupled to the distal sleeve 10, and the other end extends along the anatomical structure (for example, the blood vessels) of the body to the outside of the body, for example, to be connected to the external device. For convenience of indication, the proximal portion 20 located outside the body of the patient is not shown in the accompanying drawings. The proximal portion 20 located outside the body of the patient usually may be used as a part for doctors or the like to manipulate the blood pressure measuring catheter 1. Through manipulating this part, the blood pressure measuring catheter 1 can be pushed further to the blood vessels in the body of the patient or withdrawn from the blood vessels in the body of the patient. In addition, the proximal portion 20 located outside the body of the patient is to be connected to an external device (not shown).

In addition, in some examples, the core shaft 22 can support the proximal outer tube 21, and provide physical support for the proximal outer tube 21. In this way, it can ensure that the proximal portion 20 and the distal part 10 coupled to the proximal portion 20 can be reliably moved and pushed by moving the proximal outer tube 21 by the doctors and the like.

In some examples, materials with high hardness, such as stainless steel or hard plastics can be used for the core shaft 22. In this case, the core shaft 22 can better support the proximal outer tube 21, so that the proximal portion 20 can be better pushed. In addition, as described above, the proximal portion 20 is coupled to the distal sleeve 10. Therefore, for example, in an interventional operation of a coronary artery, doctors and the like can manipulate the proximal portion 20 located outside the body to push the distal sleeve 10 located in the body (for example, in the coronary vessel) to a predetermined position (for example, a lesion position), and can measure intravascular blood pressure by using the pressure sensor 40.

In addition, in this implementation, there is no special restriction on the shape of the proximal portion 20. In some examples, the proximal portion 20 may be in an extended elongated tube shape. In some other examples, the proximal portion 20 may alternatively be in an extended rectangular tube shape or elliptical tube shape, or the like. In this implementation, from the perspective of easy processing and facilitation of intravascular movement, the proximal portion 20 is preferably in the elongated tube shape.

In addition, there is no special restriction on the length of the proximal portion 20, as long as the length of the proximal portion 20 can be extended from the predetermined position (for example, the lesion position) in the body (for example, a vein or an artery) of the patient to a monitoring device outside the body of the patient. A typical length of the proximal portion 20 is, for example, 100 cm to 200 cm. Certainly, the length of the proximal portion 20 may alternatively be longer, for example, 300 cm, or shorter, for example, 50 cm.

### (Pressure sensor)

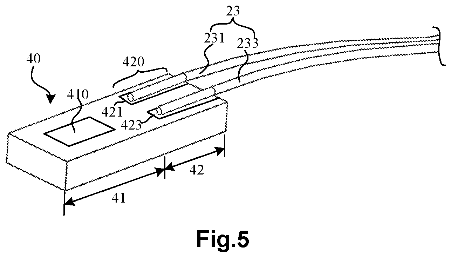

FIG. 5 is a schematic structural diagram of a pressure sensor 40 of the blood pressure measuring catheter 1.

In this implementation, the pressure sensor 40 may be a pressure sensor for measuring blood pressure (fluid pressure) in a blood vessel. As shown in FIG. 5, the pressure sensor 40 may have two output ends (referring to a bonding pad 421 and a bonding pad 422 to be described later). However, this implementation not limited thereto, and a pressure sensor 40 having more output ends may alternatively be used.

Referring to FIG. 1 or FIG. 2 again, the pressure sensor 40 may be provided on the mounting base 30. In this case, the distal sleeve 10 or the proximal portion 20 moves in the blood vessel, a stress change caused by deformation is not directly transmitted to the pressure sensor 40, so that measuring precision of the pressure sensor 40 can be improved.

In addition, the blood pressure signal generated by the pressure sensor 40 may be transmitted to the outside of the body through the conducting wire 23. In addition, in some examples, the blood pressure signal generated by the pressure sensor 40 may be transmitted to the outside of the body through a wireless connection, for example, Bluetooth, Wi-Fi, or infrared transmission.

In this implementation, as shown in FIG. 5, the pressure sensor 40 may include a sensing portion 41 and a lead portion 42. In addition, in some examples, the pressure sensor 40 may be divided into the sensing portion 41 and the lead portion 42. In other words, the pressure sensor 40 may be composed of the sensing portion 41 and the lead portion 42.

In the pressure sensor 40, the sensing portion 41 may have a sensing area 410 for sensing intravascular pressure. The pressure sensor 40 can obtain the corresponding blood pressure signal through the sensing area 410. In addition, the lead portion 42 may transmit out the blood pressure signal generated by the sensing area 410. In addition, the lead portion 42 may be electrically connected to the conducting wire 23 (referring to FIG. 1) extending along the proximal portion 20, to transmit the blood pressure signal to the outside of the body through the conducting wire 23.

In this implementation, the pressure sensor 40 may be a resistive pressure sensor, a capacitive pressure sensor, a piezoelectric pressure sensor, an inductive pressure sensor, a thermoelectric pressure sensor, or a photoelectric pressure sensor. In addition, in some examples, the pressure sensor 40 may be a pressure device using silicon as a substrate. In addition, in some examples, to restrain noise in a detection process, the pressure sensor 40 may be a differential pressure sensor.

In addition, as shown in FIG. 5, the pressure sensor 40 may be in a cuboid shape. However, in this implementation, there is no special restriction on the shape of the pressure sensor 40. For example, in some examples, the pressure sensor 40 may alternatively be in a cylindrical shape. In some other examples, the pressure sensor 40 may be in an irregular three-dimensional shape, or the like, and may be set to a corresponding shape according to a specific situation.

As described above, the sensing portion 41 has the sensing area 410 capable of sensing intravascular pressure. In some examples, for example, when the pressure sensor 40 is a piezoelectric pressure sensor, the sensing area 410 may have a sensing film capable of deformation due to stress. In this case, the sensing area 410 of the sensing portion 41 can sense the blood pressure in the blood vessels by sensing the effect of a pressure change in the blood vessels on the sensing film.

In addition, in the pressure sensor 40, the lead portion 42 is electrically connected to the sensing portion 41 (not shown), and the blood pressure signal obtained by the sensing portion 41 is transmitted out through the lead portion 42. When the pressure sensor 40 is in a cuboid shape, the connection between the lead portion 42 and the sensing portion 41 may be implemented by using an internal lead embedded in the cuboid (not shown).

In addition, the lead portion 42 is electrically connected to the conducting wire 23. Therefore, the sensing portion 41 transmits the sensed blood pressure signal outside the body through the lead portion 42 and the conducting wire 23 electrically connected to the lead portion 42. Specifically, the lead portion 42 may have a bonding pad area 420, and the conducting wire 23 may be electrically connected to the bonding pad area 420 through, for example, welding.

In this implementation, the bonding pad area 420 may include two bonding pads arranged in parallel: the bonding pad 421 and the bonding pad 423 (referring to FIG. 5). In this case, the blood pressure signal obtained by the sensing portion 41 may be transmitted to the bonding pad 421 and the bonding pad 423 through the internal lead (not shown). The bonding pad 421 and the bonding pad 423 transmit the blood pressure signal to, for example, the external device outside the body through a conducting wire 231 and a conducting wire 233 respectively. In other words, the bonding pad 421 is connected to the conducting wire 231, and the bonding pad 423 is connected to the conducting wire 233. In addition, in some examples, the bonding pad area 420 may have more bonding pads, and each bonding pad may be correspondingly welded to one conducting wire.

In addition, the conducting wire 23 may extend along the proximal portion 20. In some examples, as described above, the conducting wire 23 may extend inside the proximal outer tube 21 of the proximal portion 20. Specifically, the conducting wire 23 may be drawn from the bonding pad area 420 of the pressure sensor 40 and extends along an outer wall of the distal sleeve 10 (referring to FIG. 1), and subsequently, enters the proximal outer tube 21 of the proximal portion 20 from the junction between the distal sleeve 10 and the proximal portion 20 and extends therein. In this way, the blood pressure signal generated by the pressure sensor 40 can be transmitted to, for example, a process device (not shown) outside the body through the conducting wire 23.

In addition, in some examples, the conducting wire 23 is fixed through bonding to the distal sleeve 10 on the outer wall of the distal sleeve 10. In this case, the conducting wire 23 can be prevented from being separated from the distal sleeve 10 during pushing of the blood pressure measuring catheter 1, thereby improving reliability of welding between the conducting wire 23 and the bonding pad area 420.

### (Mounting base)

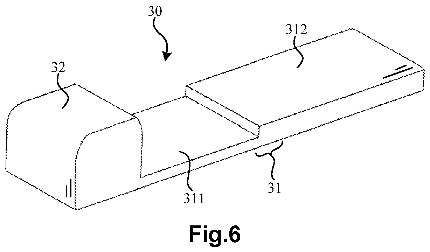

FIG. 6 is a three-dimensional schematic structural diagram of the mounting base 30 of the blood pressure measuring catheter 1.

As described above, the mounting base 30 is provided on the distal sleeve 10. The mounting base 30 may be fixed to the distal sleeve 10 through bonding or welding. The mounting base 30 is fixed on the outer surface of the distal sleeve 10. Accordingly, the mounting base 30 does not affect the receipt of the separate medical guidewire 50 by the guidewire lumen 11 of the distal sleeve 10.

As shown in FIG. 6, the mounting base 30 may be in a striped shape and roughly extends along the length direction of the distal sleeve 10. However, the mounting base 30 involved in this implementation is not limited to the foregoing shape, and may be, for example, in a block shape or an irregular shape.

In some examples, the width of the mounting base 30 may be equal to the outer diameter of the distal sleeve 10 or less than the outer diameter of the distal sleeve 10. In this way, the influence of an increase in the size of the mounting base 30 on the distal sleeve 10 can be restrained.

As described above, the pressure sensor 40 may be provided on the mounting base 30. In this implementation, preferably, the sensing portion 41 of the pressure sensor 40 and the mounting base 30 have a gap therebetween. In other words, the sensing portion 41 of the pressure sensor 40 is not in contact with the mounting base 30. In this case, the adverse effect of deformation of the mounting base 30 on the pressure sensor 40, especially the sensing portion 41 of the pressure sensor 40, can be restrained, thereby improving the measuring precision of the pressure sensor 40.

In some examples, the sensing portion 41 of the pressure sensor 40 and the bottom portion of the mounting base 30 may have a gap therebetween, and the sensing portion 41 of the pressure sensor 40 and other portions (for example, a front end) of the mounting base 30 also have a gap therebetween. Specifically, in this implementation, in the mounting base 30, the lead portion 42 of the pressure sensor 40 is fixed to the mounting base 30, the sensing portion 41 is suspended at the mounting base 30, and there is a gap between the sensing portion 41 and the mounting base 30.

In this implementation, during an interventional operation, for example, the distal sleeve 10 may be deformed due to external forces when passing through complex and varied blood vessels. In this case, because the sensing portion 41 of the pressure sensor 40 and the mounting base 30 involved in this implementation have a gap or are not in contact, for example, the adverse effect of the deformation of the distal sleeve 10 on the sensing portion 41 of the pressure sensor 40 can be effectively restrained, thereby improving the measuring precision of the pressure sensor 40.

In addition, in this implementation, the bottom portion of the mounting base 30 may be in a step shape. In other words, the mounting base 30 may have a stepped portion 31. As shown in FIG. 6, the stepped portion 31 may include a surface 311 and a surface 312, and there is a height difference between the surface 311 and the surface 312. In the stepped portion 31, along the direction away from the bottom portion of the mounting base 30, the surface 312 is higher than the surface 311. In this case, the lead portion 42 of the pressure sensor 40 may be fixed to the surface 312, and the sensing portion 41 of the pressure sensor 40 and the surface 311 have a gap therebetween.

In addition, in some examples, the Young's modulus of the mounting base 30 may be greater than the Young's modulus of the distal sleeve 10. Generally, the Young's modulus reflects a physical quantity of the deformation resistance capability of a solid material. The higher the Young's modulus, the better the deformation resistance capability of the solid material. Therefore, making the Young's modulus of the mounting base 30 greater than the Young's modulus of the distal sleeve 10 can ensure that the mounting base 30 has sufficient anti-bending strength. In this way, the mounting base 30 is not easily deformed even when the distal sleeve 10 is bent, thereby ensuring measuring precision of the pressure sensor 40 provided on the mounting base 30.

In some examples, the mounting base 30 may be composed of stainless steel, a metal alloy, or rigid engineering plastics. The metal alloy may be a cobalt-chromium alloy, a titanium alloy, a molybdenum alloy, an alloy composed of stainless steel mixed with any one of the foregoing materials, or a composite material of any one of the foregoing alloys. In addition, the rigid engineering plastics may be ABS, PMMA, PET, or PBT. Use of the foregoing materials can enable the mounting base 30 to have sufficient anti-bending strength. In this way, even if bending deformation occurs in the distal sleeve 10, stress deformation of the mounting base 30 can be effectively restrained.

In addition, in this implementation, the surface 312 may be a chamfer (not shown). In some examples, the surface 312 may be a chamfer inclined toward the proximal portion 20. In other words, along the direction away from the proximal portion 20, the surface 312 is further away from the surface 311. In this case, an average distance between the sensing area 410 of the pressure sensor 40 and the surface 311 may be increased, so that a stress effect on the sensing portion 41 of the pressure sensor 40 can be further restrained.

In addition, in some examples, the mounting base 30 may have a bottom surface configured to match the shape of the outer surface of the distal sleeve 10. In this case, the mounting base 30 may be fixed to the distal sleeve 10 by the bottom surface. In this way, the mounting base 30 can be firmly fixed to the distal sleeve 10.

In addition, the mounting base 30 has a mounting groove and a wiring groove (not shown) being disposed along the length direction of the mounting base 30 and communicating with each other. Side walls are formed on both sides of the mounting base 30 along the length direction of the mounting base 30, and the two side walls constitute the mounting groove and the wiring groove.

In this case, the pressure sensor 40 may be arranged inside the mounting groove, and the sensing portion 41 of the pressure sensor 40 and the mounting groove have a gap therebetween. Specifically, the lead portion 42 of the pressure sensor 40 may be arranged in the mounting groove, and the sensing portion 41 of the pressure sensor 40 and the surface 311 have a gap therebetween (that is, suspending in the mounting groove). In this case, the sensing portion 41 of the pressure sensor 40 may be enabled not in contact with the mounting base 30. In this way, the adverse effect of the deformation of the mounting base 30 on measuring precision of the pressure sensor 40 can be restrained.

In addition, the conducting wire 23 connected to the bonding pad area 420 of the pressure sensor 40 is drawn out through the wiring groove. In other words, the conducting wire 23 may be arranged along the wiring groove, and the wiring groove may limit the drawing direction of the conducting wire 23, so that the pressure sensor 40 can be easily provided on the mounting base 30. In addition, there is no special restriction on the shape of the mounting groove of the mounting base 30, and the mounting groove may be formed to fit the shape of the pressure sensor 40, so that the pressure sensor 40 can be easily accommodated.

In addition, the width of the mounting groove may be greater than the width of the wiring groove, the length of the mounting groove may be greater than the length of the pressure sensor 40, and the depth of the mounting groove may be greater than the thickness of the pressure sensor 40. In this way, it can ensure that the mounting groove fully accommodates the pressure sensor 40, thereby further restraining the adverse effect of stress caused by the deformation of the distal sleeve 10 or the like on the pressure sensor 40.

In addition, in this implementation, the mounting base 30 may have a front end portion 32. The front end portion 32 of the mounting base 30 closer to the foremost end of the distal sleeve 10 may extend toward the direction away from the proximal portion 20 in a tapered manner. For example, the front end portion 32 may have a surface in a circular arc shape. In this way, the transition from the front end portion 32 of the mounting base 30 to the distal sleeve 10 is smoother, thereby reducing the resistance of a blood flow during the pushing of the blood pressure measuring catheter 1 in the blood vessel, and improving operability.

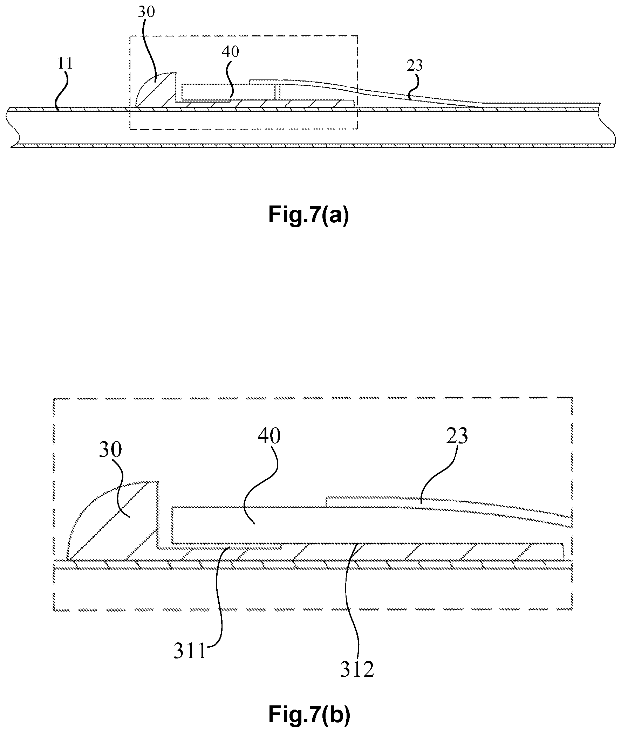

FIG. 7(a) is a partial schematic cross-sectional view of the distal part 10 of the intravascular pressure measuring catheter 1, and FIG. 7(b) is a partially enlarged schematic diagram of the intravascular pressure measuring catheter 1 having the mounting base 30 shown in FIG. 7(a).

As shown in FIG. 7(a) and FIG. 7(b), the mounting base 30 may be arranged on the outer wall of the distal sleeve 10. The pressure sensor 40 is arranged on the mounting base 30. As described above, the lead portion 42 of the pressure sensor 40 may be fixed to the surface 312 of the mounting base 30, and the sensing portion 41 of the pressure sensor 40 and the mounting base 40 have a gap therebetween. Specifically, the lead portion 42 may be fixed to the surface 312 through, for example, welding or bonding, and the sensing portion 41 of the pressure sensor 40 and the surface 311 of the mounting base 30 are in a suspension state so that there is a gap therebetween. In this case, a stress effect of the deformation of the distal sleeve 10 on the pressure sensor 40 can be restrained.

FIG. 8 is a three-dimensional schematic structural diagram illustrating that the distal sleeve of the blood pressure measuring catheter wraps the outer tube according to this implementation.

As shown in FIG. 8, the distal sleeve 10, the mounting base 30, and the pressure sensor 40 provided on the mounting base 30 may be covered by an outer tube 60. In this way, the distal sleeve 10, the mounting base 30, and the pressure sensor 40 can be protected. In addition, the outer tube 60 can provide further support for the distal sleeve 10, the mounting base 30, and the pressure sensor 40.

In addition, as shown in FIG. 8, the outer tube 60 may include a sensing opening 61 and a side opening 62. The sensing opening 61 may correspond to the sensing area 410 of the pressure sensor 40. In some examples, the sensing opening 61 may be provided on a side surface of the outer tube 60 and at a position close to the sensing area 410. In this way, the sensing area 410 of the pressure sensor 40 can sense blood pressure in the blood vessels through the sensing opening 61.

In addition, in some other examples, two symmetrical sensing openings may be provided on the side surface of the outer tube 60, so that the adverse effect of a blood flow on measurement precision of the pressure sensor 40 can be restrained. In addition, at least three sensing openings may alternatively be provided on the side surface of the outer tube 60.

In addition, the side opening 62 may correspond to the guide opening 11b of the guidewire lumen 11. In this case, the medical guidewire 50 may pass through or enter the guidewire lumen 11 through the side opening 62 of the outer tube 60.

In addition, in this implementation, there is no special restriction on the composition material of the outer tube 60, and the outer tube 60 may be optionally composed of at least one of polyester, polyamide, nylon, nylon elastomer, polyurethane, or polyimide.

Although the present invention is specifically described above with reference to the accompanying drawings and implementations, the above description is not intended to limit the present invention. The invention is defined in the appended claims.

## Claims

1. An intravascular pressure measuring catheter (1) having a mounting base (30) comprising:
a distal sleeve (10) having a guidewire lumen (11) for slidably receiving a separate medical guidewire (50);
a proximal portion (20) coupled to the distal sleeve; and
a pressure sensor (40) which is provided on the mounting base and comprises a sensing portion (41) and a lead portion (42), wherein the sensing portion has a sensing area (410) for sensing pressure, and the lead portion transmits the blood pressure signal generated by the sensing area through a conducting wire (23),
wherein the mounting base (30) is fixed to the outer surface of the distal sleeve (10),
wherein the mounting base forms side walls on both sides along the length direction of the distal sleeve, and wherein both side walls constitute respectively a mounting groove and a wiring groove communicating with each other, and wherein the pressure sensor is arranged inside the mounting groove, and the conducting wire is arranged along the wiring groove, and wherein the sensing portion of the pressure sensor and the mounting base have a gap therebetween, and wherein the Young's modulus of the mounting base (30) is greater than the Young's modulus of the distal sleeve (10).

2. The intravascular pressure measuring catheter according to claim 1, wherein the mounting base has a stepped portion (31) to which the lead portion of the pressure sensor is fixed, and wherein the sensing portion and the stepped portion have a gap therebetween.

3. The intravascular pressure measuring catheter according to claim 2, wherein the stepped portion comprises a first surface and a second surface, and there is a height difference between the first surface (311) and the second surface (312), and wherein the lead portion of the pressure sensor is fixed to the first surface, and the sensing portion of the pressure sensor and the second surface have a gap therebetween.

4. The intravascular pressure measuring catheter according to claim 1, wherein the mounting base is fixed to the distal sleeve.

5. The intravascular pressure measuring catheter according to claim 4, wherein the mounting base has a bottom surface configured to match the shape of an outer surface of the distal sleeve, and wherein the mounting base is fixed to the distal sleeve by the bottom surface.

6. The intravascular pressure measuring catheter according to claim 1, further comprising an outer tube for at least covering the mounting base, wherein a portion of the outer tube corresponding to the sensing area is provided with an opening.

7. The intravascular pressure measuring catheter according to claim 1, wherein the width of the mounting groove is greater than the width of the pressure sensor, the length of the mounting groove is greater than the length of the pressure sensor, and the depth of the mounting groove is greater than the thickness of the pressure sensor.

8. The intravascular pressure measuring catheter according to claim 1, wherein the lead portion has a bonding pad area (420), and the conducting wire is connected to the bonding pad area through welding.

9. The intravascular pressure measuring catheter according to claim 1, wherein the hardness of the mounting base is greater than the hardness of the distal sleeve.

10. The intravascular pressure measuring catheter according to claim 1, wherein the guidewire lumen of the distal sleeve comprises an extension segment (111) and a bending segment (112).

## Patentansprüche

1. Intravaskulärer Druckmesskatheter (1) mit einer Befestigungsbasis (30), umfassend:
eine distale Hülse (10) mit einem Führungsdrahtlumen (11) zum gleitenden Aufnehmen eines separaten medizinischen Führungsdrahts (50);
einen proximalen Abschnitt (20), der mit der distalen Hülse gekoppelt ist; und
einen Drucksensor (40), der an der Befestigungsbasis bereitgestellt ist und einen Erfassungsabschnitt (41) und einen Leitungsabschnitt (42) umfasst,
wobei der Erfassungsabschnitt einen Erfassungsbereich (410) zum Erfassen von Druck aufweist und der Leitungsabschnitt das vom Erfassungsbereich erzeugte Blutdrucksignal durch einen leitenden Draht (23) überträgt,
wobei die Befestigungsbasis (30) an der Außenoberfläche der distalen Hülse (10) fixiert ist,
wobei die Befestigungsbasis Seitenwände auf beiden Seiten entlang der Längsrichtung der distalen Hülse bildet, und wobei beide Seitenwände jeweils eine Befestigungsnut und eine Verdrahtungsnut bilden, die miteinander kommunizieren, und wobei der Drucksensor innerhalb der Befestigungsnut angeordnet ist und der leitende Draht entlang der Verdrahtungsnut angeordnet ist, und wobei der Erfassungsabschnitt des Drucksensors und die Befestigungsbasis einen Spalt dazwischen aufweisen, und wobei der Young-Modul der Befestigungsbasis (30) größer ist als der Young-Modul der distalen Hülse (10).

2. Intravaskulärer Druckmesskatheter nach Anspruch 1, wobei die Befestigungsbasis einen abgestuften Abschnitt (31) aufweist, an dem der Leitungsabschnitt des Drucksensors befestigt ist, und wobei der Erfassungsabschnitt und der abgestufte Abschnitt dazwischen einen Spalt aufweisen.

3. Intravaskulärer Druckmesskatheter nach Anspruch 2, wobei der abgestufte Abschnitt eine erste Oberfläche und eine zweite Oberfläche umfasst und ein Höhenunterschied zwischen der ersten Oberfläche (311) und der zweiten Oberfläche (312) besteht und wobei der Leitungsabschnitt des Drucksensors an der ersten Oberfläche befestigt ist, und der Erfassungsabschnitt des Drucksensors und die zweite Oberfläche einen Spalt dazwischen aufweisen.

4. Intravaskulärer Druckmesskatheter nach Anspruch 1, wobei die Befestigungsbasis an der distalen Hülse befestigt ist.

5. Intravaskulärer Druckmesskatheter nach Anspruch 4, wobei die Befestigungsbasis eine Bodenoberfläche aufweist, die so konfiguriert ist, dass sie der Form einer Außenoberfläche der distalen Hülse entspricht, und wobei die Befestigungsbasis an der distalen Hülse durch die Bodenoberfläche befestigt ist.

6. Intravaskulärer Druckmesskatheter nach Anspruch 1, ferner umfassend ein Außenrohr, um mindestens die Befestigungsbasis abzudecken, wobei ein Abschnitt des Außenrohrs, der dem Erfassungsbereich entspricht, mit einer Öffnung versehen ist.

7. Intravaskulärer Druckmesskatheter nach Anspruch 1, wobei die Breite der Befestigungsnut größer als die Breite des Drucksensors ist, die Länge der Befestigungsnut größer als die Länge des Drucksensors ist und die Tiefe der Befestigungsnut größer als die Dicke des Drucksensors ist.

8. Intravaskulärer Druckmesskatheter nach Anspruch 1, wobei der Leitungsabschnitt einen Bonding-Pad-Bereich (420) aufweist und der leitende Draht mit dem Bonding-Pad-Bereich durch Schweißen verbunden ist.

9. Intravaskulärer Druckmesskatheter nach Anspruch 1, wobei die Härte der Befestigungsbasis größer ist als die Härte der distalen Hülse.

10. Intravaskulärer Druckmesskatheter nach Anspruch 1, wobei das Führungsdrahtlumen der distalen Hülse ein Verlängerungssegment (111) und ein Biegesegment (112) umfasst.

## Revendications

1. Cathéter de mesure de pression intravasculaire (1) comportant une base de montage (30) comprenant :
un manchon distal (10) qui comporte une lumière de fil-guide (11) pour recevoir de manière coulissante un fil-guide médical séparé (50) ;
une partie proximale (20) couplée au manchon distal ; et
un capteur de pression (40) qui est fourni sur la base de montage et comprend une partie de détection (41) et une partie de connexion (42),
dans lequel la partie de détection comporte une zone de détection (410) pour détecter la pression, et la partie de connexion transmet le signal de pression sanguine généré par la zone de détection à travers un fil conducteur (23),
dans lequel la base de montage (30) est fixée à la surface extérieure du manchon distal (10),
dans lequel la base de montage forme des parois latérales sur les deux côtés le long de la direction de la longueur du manchon distal, et dans lequel les deux parois latérales constituent respectivement une rainure de montage et une rainure de câblage communiquant l'une avec l'autre, et dans lequel le capteur de pression est agencé à l'intérieur de la rainure de montage, et le fil conducteur est agencé le long de la rainure de câblage, et dans lequel la partie de détection du capteur de pression et la base de montage ont un espace entre elles, et dans lequel le module de Young de la base de montage (30) est supérieur au module de Young du manchon distal (10).

2. Cathéter de mesure de pression intravasculaire selon la revendication 1, dans lequel la base de montage a une partie étagée (31) à laquelle la partie de connexion du capteur de pression est fixée, et dans lequel la partie de détection et la partie étagée ont un espace entre elles.

3. Cathéter de mesure de pression intravasculaire selon la revendication 2, dans lequel la partie étagée comprend une première surface et une seconde surface, et il existe une différence de hauteur entre la première surface (311) et la seconde surface (312), et dans lequel la partie de connexion du capteur de pression est fixée à la première surface, et la partie de détection du capteur de pression et la seconde surface ont un espace entre elles.

4. Cathéter de mesure de pression intravasculaire selon la revendication 1, dans lequel la base de montage est fixée au manchon distal.

5. Cathéter de mesure de pression intravasculaire selon la revendication 4, dans lequel la base de montage a une surface inférieure configurée pour épouser la forme d'une surface extérieure du manchon distal, et dans lequel la base de montage est fixée au manchon distal par la surface inférieure.

6. Cathéter de mesure de pression intravasculaire selon la revendication 1, comprenant en outre un tube extérieur pour recouvrir au moins la base de montage, dans lequel une partie du tube extérieur correspondant à la zone de détection est pourvue d'une ouverture.

7. Cathéter de mesure de pression intravasculaire selon la revendication 1, dans lequel la largeur de la rainure de montage est supérieure à la largeur du capteur de pression, la longueur de la rainure de montage est supérieure à la longueur du capteur de pression, et la profondeur de la rainure de montage est supérieure à l'épaisseur du capteur de pression.

8. Cathéter de mesure de pression intravasculaire selon la revendication 1, dans lequel la partie de connexion a une zone de plot de liaison (420), et le fil conducteur est connecté à la zone de plot de liaison par soudage.

9. Cathéter de mesure de pression intravasculaire selon la revendication 1, dans lequel la dureté de la base de montage est supérieure à la dureté du manchon distal.

10. Cathéter de mesure de pression intravasculaire selon la revendication 1, dans lequel la lumière de fil-guide du manchon distal comprend un segment d'extension (111) et un segment de flexion (112).
